(19) Europäisches Patentamt
European Patent Office
Office européen des brevets

(11) **EP 2 626 717 A1**

(12) **EUROPEAN PATENT APPLICATION**

(43) Date of publication:
**14.08.2013 Bulletin 2013/33**

(51) Int Cl.:
***G01R 33/36*** *(2006.01)*

(21) Application number: **13154627.7**

(22) Date of filing: **08.02.2013**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **08.02.2012 KR 20120012725**

(71) Applicant: **Samsung Electronics Co., Ltd
Gyeonggi-do 443-742 (KR)**

(72) Inventors:
• **Yi, Yong Seok
Gyeonggi-do (KR)**
• **Kwon, Young Cheol
Gyeonggi-do (KR)**
• **Kim, Joon Soo
Seoul (KR)**
• **Cho, Hyug Rae
Seoul (KR)**

(74) Representative: **Grünecker, Kinkeldey,
Stockmair & Schwanhäusser
Leopoldstrasse 4
80802 München (DE)**

(54) **Magnetic resonance imaging apparatus**

(57)    The invention relates to a magnetic resonance imaging apparatus that is provided with an RF receiver comprising an RF receiving coil, a preamplifier and a power producing module configured to produce power that is to be supplied to the RF receiver. The power producing module is energized through an RF pulse which is applied to a subject from an RF transmitting coil of the magnetic resonance imaging apparatus.

FIG. 4

EP 2 626 717 A1

## Description

BACKGROUND

### 1. Field

Exemplary embodiments of the present disclosure relate to a magnetic resonance imaging apparatus configured to be used to diagnose of various diseases by using a magnetic resonance image.

### 2. Description of the Related Art

In general, a medical imaging apparatus is an apparatus configured to provide an image by obtaining information of a patient. The medical imaging apparatuses include an x-ray apparatus, an ultrasound wave diagnostic apparatus, a computerized tomography apparatus, and a magnetic resonance imaging apparatus. Among the above medical imaging apparatuses, the magnetic resonance imaging apparatus provides images having a variety of diagnostic information and superior contrast while permitting a relatively free condition for the image photographing, thereby taking an important position in medical image diagnostics.

A Magnetic Resonance Imaging (MRI) refers to obtaining an image of the density and the physiochemical characteristic of atomic nuclei by generating a nuclear magnetic resonance phenomenon in a hydrogen atomic nucleus by use of a magnetic field, which is not harmful to a human body, and radio frequency (RF), which is a non- ionizing radiation.

In detail, the magnetic resonance imaging apparatus is an image diagnostic apparatus configured to be used to diagnose an inside of a human body by supplying a constant frequency and energy to the atomic nuclei whereby the atomic nucleus is applied with a constant magnetic field and the energy emitted from the atomic nucleus is converted into a signal.

Since a proton composing the atomic nucleus is provided with spin angular momentum and a magnetic dipole, when a magnetic field is applied to the proton, the proton is aligned in a direction of the magnetic field, and the atomic nucleus performs a precession with respect to the direction of the magnetic field. By the precession as such, an image of a human body may be obtained through a nuclear magnetic resonance phenomenon.

SUMMARY

Therefore, it is an aspect of the present disclosure to provide a magnetic resonance imaging apparatus provided with an RF receiving part having a spectrometer configured to perform a digital signal processing by demodulating a magnetic resonance signal into a baseband, and a power producing module configured to produce power that is to be supplied to the RF receiving part, by use of an RF pulse which is applied to a subject from an RF transmitting coil.

Additional aspects of the disclosure will be set forth in part in the description which follows and, in part, will be obvious from the description, or may be learned by practice of the disclosure.

In accordance with one aspect of the present disclosure, a radio frequency (RF) receiving part of a magnetic resonance imaging apparatus having a magnet assembly, the RF receiving part includes an RF receiving coil, an RF amplifier and a power producing module. The RF receiving coil may be configured to receive a magnetic resonance signal generated from a subject. The RF amplifier may be configured to amplify the magnetic resonance signal received from the RF receiving coil. The power producing module may be configured to produce power by receiving an RF pulse applied from an RF transmitting coil of the magnet assembly.

The power producing module may be configured to supply the power to the RF amplifier.

The power producing module may include a power coil, a power storing part and a power supply adjusting part. The power coil may be configured to produce power by receiving the RF pulse applied from the RF transmitting coil part. The power storing part may be configured to store the power produced from the power coil. The power supply adjusting part may be configured to adjust a supply of the power stored in the power storing part.

The RF receiving part may further include a spectrometer that may be configured to perform a digital signal processing on the signal that is amplified by the RF amplifier. The spectrometer may include an Analog-Digital (AD) converter. The AD converter may be configured to convert the signal amplified by the RF amplifier into a digital signal. The processor may be configured to convert the digital signal, which is converted by the AD converter, into a baseband signal through modulation.

The processor may be configured to adjust the supply of power to the RF amplifier by controlling the power producing module.

The processor may be configured to control a time of the supply of power by the power supply adjusting part provided at the power producing module, so that the power is supplied to the RF amplifier when the magnetic resonance signal is amplified by the RF amplifier.

In accordance with another aspect of the present disclosure, a magnetic resonance apparatus includes an RF transmitting coil part, and an RF receiving part. The RF transmitting coil part may be configured to apply an RF pulse to a subject. The RF receiving part may be configured to receive a magnetic resonance signal generated from the subject, and provided with a power producing module configured to produce power by receiving the RF pulse applied from the RF transmitting coil part.

The power producing module may include a power coil, a power storing part, and a power supply adjusting part. The power coil may be configured to produce power by receiving the RF pulse that is applied from the RF transmitting coil part. The power storing part may be configured to store the power produced by the power coil. The power supply adjusting part may be configured to adjust a supply of the power stored in the power storing part.

The power produced from the power producing module may be transmitted to each part that composes the RF receiving part.

The RF receiving part may include an RF receiving coil, an RF amplifier and a spectrometer. The RF receiving coil may be configured to receive a magnetic resonance signal that is generated from the subject. The RF amplifier may be configured to amplify the magnetic resonance signal that is received from the RF receiving coil. The spectrometer may be configured to perform a digital signal processing on the signal that is amplified by the RF amplifier.

The spectrometer may include an Analog-Digital (AD) converter, and a processor. The AD converter may be configured to convert the signal amplified by the RF amplifier into a digital signal. The processor may be configured to convert the digital signal, which is converted by the AD converter, into a baseband signal through demodulation.

The magnetic resonance apparatus may further include a transmitting/receiving part. The transmitting/receiving part may be configured to transmit the signal, which has been subject to the digital signal processing by the spectrometer, to a computer system in a wireless manner.

A magnetic resonance imaging apparatus in accordance with the aspect of the present disclosure is provided with a spectrometer included at an RF receiving part, so that a SNR (Signal-to-Noise Ratio) may be enhanced, and thus the quality of an image may be enhanced.

In one exemplary embodiment, there is a radio frequency (RF) receiver of a magnetic resonance imaging apparatus having a magnet assembly, the RF receiver including: an RF receiving coil configured to receive a magnetic resonance signal generated from a subject; an RF amplifier configured to amplify the magnetic resonance signal received from the RF receiving coil; and a power generator configured to generate power by receiving an RF pulse output from an RF coil assembly of the magnet assembly.

In another exemplary embodiment, there is a magnetic resonance apparatus including: a radio frequency (RF) transmitting coil part configured to apply an RF pulse to a subject; and an RF receiver configured to receive a magnetic resonance signal generated from the subject, and provided with a power generator configured to generate power by receiving the RF pulse output from the RF assembly.

In one exemplary embodiment, there is a radio frequency (RF) receiver of a magnetic resonance imaging apparatus which outputs an RF pulse through an RF transmitting coil, the RF receiver including: an RF coil which receives a magnetic resonance signal; a preamplifier which amplifies the received magnetic resonance signal; and a power generator which receives an RF pulse and converts the received RF pulse into power and outputs the power to the preamplifier.

The power generator may include: a coil which receives the RF pulse and converts the received RF pulse into power; a power storage which stores the power; and a power output which outputs the stored power to the preamplifier.

The RF receiver may further include a processor which controls the power output so that the power is supplied to the preamplifier during an operation of the preamplifier amplifying the received magnetic resonance signal.

BRIEF DESCRIPTION OF THE DRAWINGS

These and/or other aspects of the disclosure will become apparent and more readily appreciated from the following description of the exemplary embodiments, taken in conjunction with the accompanying drawings of which:

FIG. 1 is a block diagram of a magnetic resonance imaging apparatus in accordance with one exemplary embodiment of the present disclosure.
FIG. 2 is a drawing showing an exterior appearance of a magnetic resonance imaging apparatus in accordance with one exemplary embodiment of the present disclosure.
FIG. 3 is a drawing a space, at which a subject is placed, divided by an x-axis, a y-axis, and a z-axis.
FIGS. 4 and 5 are drawings illustrating a structure of an RF receiving part of FIG. 1.

DETAILED DESCRIPTION

Reference will now be made in detail to the exemplary embodiments of the present disclosure, examples of which are illustrated in the accompanying drawings, wherein like reference numerals refer to like elements throughout.

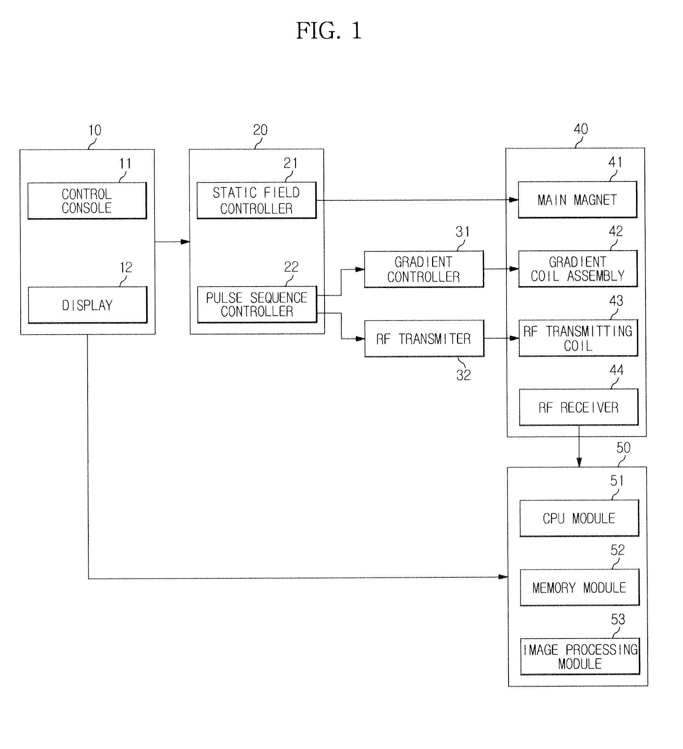

FIG. 1 is a block diagram of a magnetic resonance imaging apparatus in accordance with one exemplary embodiment of the present disclosure.

Referring to FIG. 1, a magnetic resonance imaging apparatus in accordance with one exemplary embodiment of the present disclosure includes a magnet assembly 40, a controller 20 to control the operation of the magnet assembly 40, a terminal 10, e.g., a user manipulation part, and a computer system 50.

The magnet assembly 40 includes a main magnet 41, i.e., a static magnetic field unit, forming a static magnetic field, i.e., a B0 magnetic field, therein, a gradient coil assembly 42, i.e., gradient coils, which form a gradient in the static magnetic field, an RF transmitting coil assembly 43 to apply an RF pulse to excite the atomic nuclei, and an RF receiver 44, e.g., an RF receiving coil, to receive a magnetic resonance signal from the atomic nuclei.

The controller 20 includes a static field controller 21 configured to control the intensity and the direction of the static magnetic field that is formed by the main magnet 41, and a pulse sequence controller 22 configured to design, i.e., to form, a pulse sequence and to control the gradient coil assembly 42 and the RF coil assembly 43 according to the designed pulse sequence.

The magnetic resonance imaging apparatus in accordance with one exemplary embodiment of the present disclosure includes a gradient controller 31 to apply a gradient signal to the gradient coil assembly 42 and an RF transmitter 32 to apply an RF signal to the RF coil assembly 43, so that the gradient formed on the static magnetic field and the RF pulse applied to the atomic nuclei are adjusted as the pulse sequence controller 22 controls the gradient controller 31 and the RF transmitter 32.

The terminal 10 may receive a control command from the user, regarding the overall operations of the magnetic resonance imaging apparatus and particularly, receiver a command with respect to a scan sequence, and the pulse sequence may be generated accordingly.

The terminal 10 may include a control console 11 provided for an administrator to manipulate a system, and a display 12 configured for a user to diagnose the health condition of a subject 200 by displaying a control state and a magnetic resonance image. The terminal 10 is connected to the computer system 50 through a link.

The computer system 50 may include a plurality of modules communicating with each other through a backplane. The plurality of modules includes an image processing module 53, a memory module 52, and a CPU module 51. The computer system 50 may be linked to a disc memory apparatus and to a tape drive that have a memory of image data and a program.

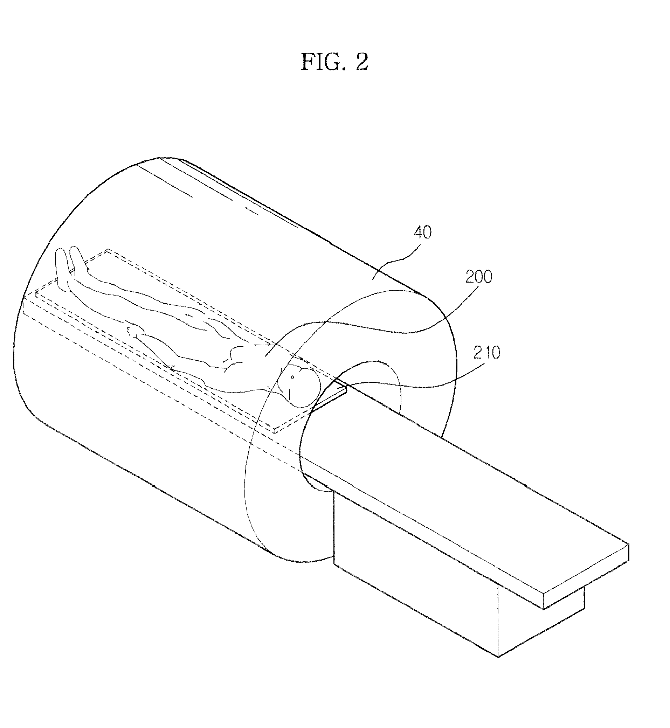

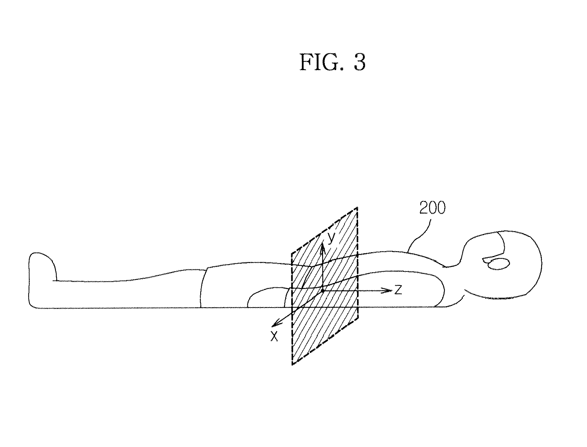

FIG. 2 is a drawing showing an exterior appearance of a magnetic resonance imaging apparatus in accordance with one exemplary embodiment of the present disclosure, and FIG. 3 is a drawing a space, at which a subject is placed, divided by an x-axis, a y-axis, and a z-axis.

Referring to FIG. 2, the magnet assembly 40 is provided with a cylindrical shape having an inside space thereof empty, and the inside space is referred to as a cavity part. The subject 200 lying on a transfer part 210, e.g., patient table, is placed into the cavity part to obtain a magnetic resonance signal.

As explained in FIG. 1, the magnetic assembly 40 includes the main magnet 41, the gradient coil assembly 42, the RF transmitting coil assembly 43, and the RF receiver 44. The main magnet 41 may be provided in a form having a coil surrounding the circumference of the cavity part, and when current is applied to the main magnet 41, a constant static magnetic field is formed inside the magnet assembly 40, that is, at the cavity part, while the direction of the static magnetic field is generally in parallel to the coaxial direction of the magnet assembly 40.

When a static magnetic field is formed at the cavity part, the atoms of the subject, that is, the nuclei of hydrogen atoms, are aligned in the direction of the static magnetic field, and performs a precession around the direction of the static magnetic field. The precession speed of the atomic nucleus may be expressed as a precession frequency, and the frequency as such is referred to as a Larmor frequency that may be expressed as the [EQN. 1] below:

$$[EQN.\ 1]$$

$$\omega = \gamma B0$$

Here, the $\omega$ is referred to as the Larmor frequency, the $\gamma$ is referred to as the proportional constant, and the B0 is referred to as the intensity of an external magnetic field. The proportional constant is varied according to the type of an atomic nucleus, the unit of the intensity of the external magnetic field is Tesla T or Gauss G, and the unit of the precession frequency is Hz.

For example, the hydrogen proton is provided with the precession frequency of about 42.58MHz in the external magnetic field of 1T, and since the element that takes up the largest proportion among all the elements of a human body is hydrogen, a MRI obtains a magnetic resonance signal by using the precession of the hydrogen proton. The gradient coil assembly 42, by generating a gradient at the static magnetic field formed at the cavity part, forms a gradient magnetic field.

As illustrated on FIG. 3, the axis, which is parallel to a direction lengthwise from the head and the feet of the subject 200, that is, the axis that is parallel to the direction of the static magnetic field may be set as the z-axis, the axis, which is parallel to a direction widthwise along of the subject 200, may be set as the x-axis, and the axis, which is parallel to the vertical direction, may be set as the y-axis.

In order to obtain three-dimensional space information, the gradient magnetic fields for each of the x-axis, the y-axis, and the z-axis are needed, and thus the gradient coil assembly 42 includes three pairs of gradient coils. The z-axis gradient coil forms a gradient in the z-axis direction, and as the current applied to the z-axis gradient coil becomes stronger, the gradient magnetic field having larger gradient is formed, and the forming of the gradient magnetic field having larger gradient enables the selection of a thin slice. Thus, the z-axis gradient coil is used for the selection of the slice.

The gradient magnetic field generated by the x-axis gradient coil is configured to provide spatial position of the subject 200 in the x-axis, and the gradient magnetic field is used for a frequency encoding. In addition, the gradient magnetic field generated by the y-axis gradient coil is mainly used for a phase encoding.

The gradient coil assembly 42 is connected to the gradient controller 31, and the gradient controller 31 is configured to apply a driving signal to the gradient coil assembly 42 according to the control signal transmitted from the pulse sequence controller 22 to generate a gradient magnetic field. The gradient controller 31, by controlling the three gradient coils of the gradient coil assembly 42, may be provided with three driving circuits.

As described above, the atomic nuclei aligned by the external magnetic field perform precession at the Larmor frequency, and a vector sum of a magnetization of a plurality of atomic nuclei may be expressed as a net magnetization M.

The component of the z-axis of the net magnetization M is difficult to be measured, and only Mxy may be detected. Thus, in order to obtain a magnetic resonance signal, the net magnetization M is needed to be present on the XY plane surface, and the such is referred to as the excitation of an atomic nucleus, and in order to excite the atomic nucleus, the RF pulse tuned at the Larmor frequency of the atomic nucleus is needed to be applied to the static magnetic field.

The RF transmitting coil assembly 43, in order to excite the atomic nucleus at an inside the subject 200, generates radio-frequency magnetic field at a static magnetic field space. The generating of the radio-frequency magnetic field is hereinafter referred to as the application of the RF pulse.

The RF transmitting coil assembly 43 is connected to the RF transmitter 32, and the RF transmitter 32, according to the control signal transmitted from the pulse sequence controller 22, applies a driving signal to the RF transmitting coil assembly 43 to emit an RF pulse.

The RF transmitter 32 may include a modulation circuit and configured to modulate a radio-frequency output signal into a pulse signal, and an RF power amplifier configured to amplify the pulse signal.

As a method mainly for the purpose of obtaining a magnetic resonance signal from an atomic nucleus, a spin echo pulse sequence is present. When the RF transmitting coil assembly 43 applies an RF pulse, after a first RF pulse is applied, if an RF pulse is applied one more time at a time interval of $\Delta t$, a strong transverse magnetization occurs at the atomic nuclei after another time interval of $\Delta t$, and from such, a magnetic resonance signal may be able to be obtained. The above is referred to as the spin echo pulse sequence, and the time taken for the magnetic resonance signal to occur after the first RF pulse is applied is referred to as the Time Echo 'TE'.

The degree of the proton being flipped may be expressed by the angle of movement of the proton from the axis that the proton was positioned before being flipped, and by the degree of the flip, the degree of the proton being flipped may be expressed as a 90°RF pulse and 180 °RF pulse.

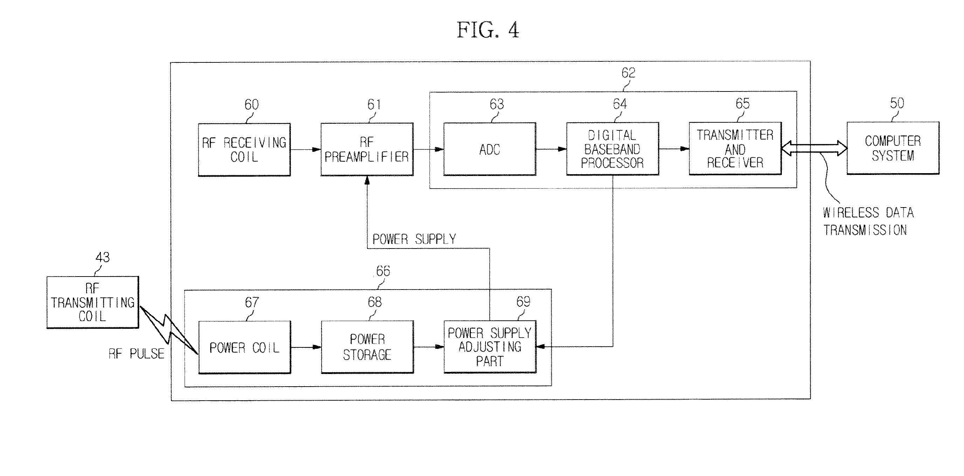

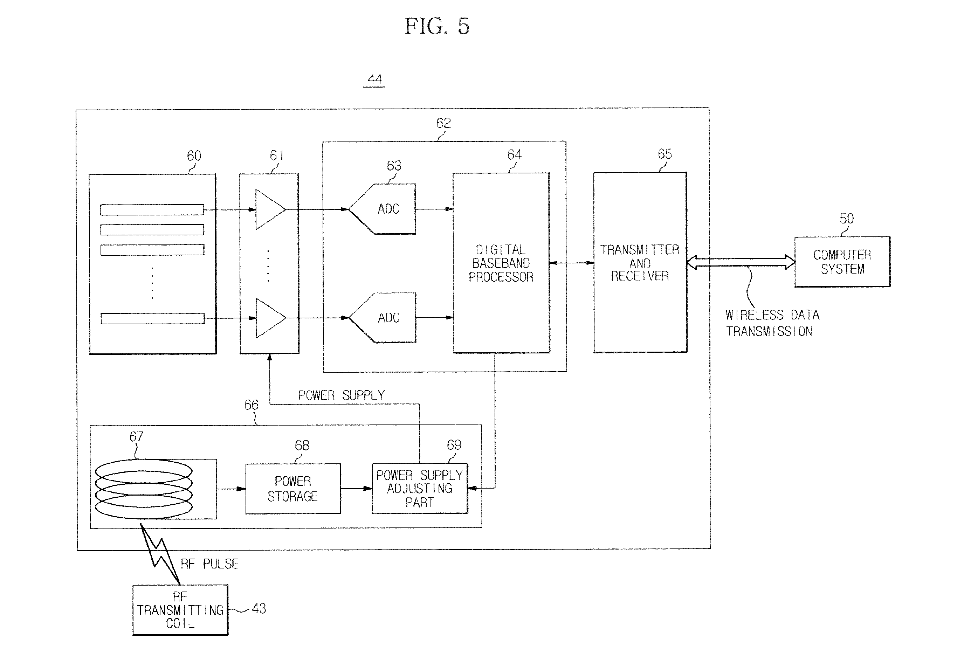

FIGS. 4 and 5 are drawings illustrating a structure of the RF receiver 44 of FIG. 1. The RF receiver 44 is configured to receive a magnetic resonance signal that an atomic nucleus emits.

Referring to FIGS. 4 and 5, the RF receiver 44 includes an RF receiving coil 60 configured to receive a magnetic resonance signal that an atomic nucleus emits, and an RF preamplifier 61 configured to amplify the magnetic resonance signal that is received from the RF receiving coil 60. Since the magnetic resonance signal that an atomic nucleus emits is weak, the magnetic resonance signal is subject to a signal processing process after being amplified by about 50dB and 100dB at the RF preamplifier 61.

In addition, the RF receiver 44 includes a spectrometer 62 configured to perform a digital signal processing on the signal that is amplified through the RF preamplifier 61.

The spectrometer 62 includes an AD converter (Analog-to-Digital Converter) 63 configured to convert the signal, which is amplified through the RF preamplifier 61, into the digital signal that may be processed by a computer, and a digital baseband processor 64 configured to convert the magnetic resonance signal of the RF band into the baseband signal by modulating the magnetic resonance signal.

The signal being transmitted to the spectrometer 62 through the RF preamplifier 61 may be transmitted via a cable communication method or a wireless communication method.

The signal converted into the broadband signal through the spectrometer 62 as such is transmitted to the computer system 50 after passing through the transmitter and receiver 65, e.g., transmitting/receiving part, in a wireless manner.

In addition, the RF receiver 44 includes a power producing module 66 which is implemented in hardware in an exemplary embodiment.

The power producing part 66, i.e., power generator, may include a power coil 67 configured to receive the RF pulse output by the RF coil assembly 43 to generate power, a power storage 68 configured to store the power produced by the power coil 67, and a power supply adjusting part 69 (implemented in hardware in an exemplary embodiment) configured to deliver the power stored in the power storing part 68 to each component of the RF receiver 44 and to control each component of the RF. The power coil 67 is configured to produce power by receiving the RF pulse emitted to the subject 200 to excite an atomic nucleus.

That is, without receiving the energy needed for the production of power separately from an outside, the power is produced by using the RF pulse that is applied to the subject 200 from the RF coil assembly 43, which is an existing component of the magnetic resonance imaging apparatus.

The power produced by the power coil 67 is stored in the power storage 68, and the power storage 68 may be implemented by employing various configurations that are generally known in the art.

The power supply adjusting part 69, in order to deliver the power stored at the power storage 68 to each component of the RF receiver 44, adjusts the supply of the power. The driving of the power supply adjusting part 69 may be adjusted by the digital baseband processor 64. The power produced by the power producing module 66 is mainly supplied to the RF preamplifier 61 at which a large amount of power is consumed.

The digital baseband processor 64, may adjust the supply of power being supplied to the RF preamplifier 61 by controlling the time of the supply of power by the power supply adjusting part 69 such that the power is supplied to the RF preamplifier 61 when the magnetic resonance signal is amplified by the RF preamplifier 61. Hereinafter, the operation of the RF receiver 44 having the power producing module 66 will be described.

When the RF pulse is applied to the subject 200 by the RF transmitting coil 43 to excite the atomic nuclei, the RF pulse excites the atomic nuclei while a portion of the RF pulse is received by the power coil 67 provided at the power producing module 66 of the RF receiver 44, and the received RF pulse by the power coil 67 is used for the power production.

The power produced by the power coil 67 is stored in the power storage 68.

The RF receiving coil 60 of the RF receiver 44 receives the magnetic resonance signal being emitted from an atomic nucleus that is excited by the RF pulse, and the RF preamplifier 61 amplifies the magnetic resonance signal that is received by the RF receiving coil 60. At this time, the power supply adjusting part 69 of the power producing module 66 supplies power to the RF preamplifier 61, so that the weak magnetic resonance signal may be amplified.

The signal amplified by the RF preamplifier 61 is converted into the digital signal by being transmitted to the AD converter 63, and through the digital baseband processor 64, the magnetic resonance signal of the RF band is demodulated into the baseband signal. The signal having subject to the digital signal processing process as such is transmitted to the computer system 50 through a transmitter and receiver 65 in a wireless manner.

Although a few exemplary embodiments of the present disclosure have been shown and described, it would be appreciated by those skilled in the art that changes may be made in these embodiments without departing from the principles and spirit of the disclosure, the scope of which is defined in the claims and their equivalents.

**Claims**

1. A radio frequency (RF) receiver of a magnetic resonance imaging apparatus having a magnet assembly, the RF receiver comprising:

    an RF receiving coil configured to receive a magnetic resonance signal generated from a subject;
    an RF amplifier configured to amplify the magnetic resonance signal received from the RF receiving coil; and
    a power generator configured to generate power by receiving an RF pulse output from an RF coil assembly of the magnet assembly.

2. The RF receiver of claim 1, wherein the power generator is configured to supply the power to the RF amplifier.

3. The RF receiver of claim 1, wherein the power generator comprises:

    a power coil configured to generate the power by receiving the RF pulse output from the RF coil assembly;
    a power storage configured to store the power produced from the power coil; and
    a power supply adjusting part configured to adjust a supply of the power stored in the power storing part.

4. The RF receiver of claim 1, further comprising:

    a spectrometer configured to perform a digital signal processing on the amplified magnetic resonance signal.

5. The RF receiver of claim 4, wherein the spectrometer comprises:

    an Analog-Digital (AD) converter configured to convert the amplified magnetic resonance signal into a digital signal; and
    a processor configured to convert the digital signal into a baseband signal through modulation.

6. The RF receiver of claim 5, wherein the processor is further configured to adjust the supply of power to the RF amplifier by controlling the power generator.

7. The RF receiver of claim 6, wherein the processor is configured to control a time of the supply of power by the power supply adjusting part provided at the power generator, so that the power is supplied to the RF amplifier when the magnetic resonance signal is amplified by the RF amplifier.

8. A magnetic resonance apparatus comprising:

    a radio frequency (RF) transmitting coil part configured to apply an RF pulse to a subject; and
    an RF receiver configured to receive a magnetic resonance signal generated from the subject, and provided with a power generator configured to generate power by receiving the RF pulse output from the RF coil assembly.

9. The magnetic resonance apparatus of claim 8, wherein the power generator comprises:

a power coil configured to generate the power by receiving the RF pulse that is output from the RF coil assembly;
a power storage configured to store the power produced by the power coil; and
a power supply adjusting part configured to adjust a supply of the power stored in the power storing part.

10. The magnetic resonance apparatus of claim 9, wherein the power generated from the power generator is transmitted to an RF preamplifier of the RF receiver.

11. The magnetic resonance apparatus of claim 8, wherein the RF receiver comprises:

an RF receiving coil configured to receive a magnetic resonance signal that is generated from the subject;
an RF amplifier configured to amplify the magnetic resonance signal that is received from the RF receiving coil; and
a spectrometer configured to perform a digital signal processing on the amplified magnetic resonance signal.

12. The magnetic resonance apparatus of claim 11, wherein the spectrometer comprises:

an Analog-Digital (AD) converter configured to convert the amplified magnetic resonance signal into a digital signal; and
a processor configured to convert the digital signal into a baseband signal through demodulation.

13. The magnetic resonance apparatus of claim 11, further comprising:

a transmitter and receiver configured to transmit the digital signal to a computer system in a wireless manner.

14. A radio frequency (RF) receiver of a magnetic resonance imaging apparatus which outputs an RF pulse through an RF transmitting coil, the RF receiver comprising:

an RF coil which receives a magnetic resonance signal;

a preamplifier which amplifies the received magnetic resonance signal; and
a power generator which receives an RF pulse and converts the received RF pulse into power and outputs the power to the preamplifier.

15. The RF receiver of claim 14, wherein the power generator comprises:

a coil which receives the RF pulse and converts the received RF pulse into power;
a power storage which stores the power; and
a power output which outputs the stored power to the preamplifier.

16. The RF receiver of claim 15 further comprising a processor which controls the power output so that the power is supplied to the preamplifier during an operation of the preamplifier amplifying the received magnetic resonance signal.

FIG. 1

FIG. 2

FIG. 3

FIG. 4

EP 2 626 717 A1

# FIG. 5

EP 2 626 717 A1

Europäisches
Patentamt

European
Patent Office

Office européen
des brevets

# EUROPEAN SEARCH REPORT

Application Number

EP 13 15 4627

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/226841 A1 (BOSKAMP EDDY B [US] ET AL) 12 October 2006 (2006-10-12) * paragraphs [0018] - [0031]; figures 1,3,4 * | 1-16 | INV. G01R33/36 |
| X | WO 2006/000928 A2 (KONINKL PHILIPS ELECTRONICS NV [NL]; PHILIPS CORP [US]; LUEDEKE KAI-MI) 5 January 2006 (2006-01-05) * page 1, line 7 - page 1, line 22 * * page 4, line 4 - page 9, line 17 * * page 10, line 1 - page 11, line 2 * * figures 1-5 * | 1-5,8-16 | |
| X | WO 2004/089211 A2 (KONINKL PHILIPS ELECTRONICS NV [NL]; TUITHOF HANS H [NL]; DEN BOEF JOH) 21 October 2004 (2004-10-21) * page 5, line 6 - page 13, line 9; figures 2,4,5 * | 1-5,8-16 | |
| X | WEI ET AL: "A realization of digital wireless transmission for MRI signals based on 802.11b", JOURNAL OF MAGNETIC RESONANCE, ACADEMIC PRESS, ORLANDO, FL, US, vol. 186, no. 2, 8 June 2007 (2007-06-08), pages 358-363, XP022107127, ISSN: 1090-7807, DOI: 10.1016/J.JMR.2007.03.003 * the whole document * | 1-5,8-16 | TECHNICAL FIELDS SEARCHED (IPC) G01R |
| X | US 5 245 288 A (LEUSSLER CHRISTOPH G [DE]) 14 September 1993 (1993-09-14) * column 2, line 66 - column 4, line 50 * * column 6, line 14 - column 6, line 57 * * figures 1-3 * | 1-5, 8-12, 14-16 | |

-/--

The present search report has been drawn up for all claims

| Place of search | Date of completion of the search | Examiner |
|---|---|---|
| Munich | 22 April 2013 | Lersch, Wilhelm |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

...............................................................................................

& : member of the same patent family, corresponding
document

EPO FORM 1503 03.82 (P04C01)

Europäisches
Patentamt

European
Patent Office

Office européen
des brevets

# EUROPEAN SEARCH REPORT

Application Number

EP 13 15 4627

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/067682 A2 (KONINKL PHILIPS ELECTRONICS NV [NL]; VAN HELVOORT MARINUS J A M [NL];) 29 June 2006 (2006-06-29) * page 2, line 5 - page 5, line 20 * * page 6, line 17 - page 8, line 14 * * figures 1,2 * | 1-16 | |
| A | WO 2008/001326 A1 (KONINKL PHILIPS ELECTRONICS NV [NL]; HARVEY PAUL ROYSTON [GB]) 3 January 2008 (2008-01-03) * page 5, line 18 - page 6, line 8 * * page 7, line 21 - page 8, line 5 * * page 9, line 15 - page 9, line 22 * * figures 3,4 * | 5,12 | |
| A | US 2009/058420 A1 (ADACHI KOHEI [JP] ET AL) 5 March 2009 (2009-03-05) * paragraphs [0022] - [0031], [0040] - [0061], [0082] - [0099]; figures 1-3,6,7 * | 6,7,16 | |
| | | | TECHNICAL FIELDS SEARCHED (IPC) |

The present search report has been drawn up for all claims

| Place of search | Date of completion of the search | Examiner |
|---|---|---|
| Munich | 22 April 2013 | Lersch, Wilhelm |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

...................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 13 15 4627

This annex lists the patent family members relating to the patent documents cited in the above-mentioned European search report.
The members are as contained in the European Patent Office EDP file on
The European Patent Office is in no way liable for these particulars which are merely given for the purpose of information.

22-04-2013

| Patent document cited in search report | | Publication date | Patent family member(s) | | Publication date |
|---|---|---|---|---|---|
| US 2006226841 | A1 | 12-10-2006 | US | 2006226841 A1 | 12-10-2006 |
| | | | US | 2008048660 A1 | 28-02-2008 |
| WO 2006000928 | A2 | 05-01-2006 | CN | 101014870 A | 08-08-2007 |
| | | | EP | 1761791 A2 | 14-03-2007 |
| | | | JP | 2008503298 A | 07-02-2008 |
| | | | US | 2008272786 A1 | 06-11-2008 |
| | | | WO | 2006000928 A2 | 05-01-2006 |
| WO 2004089211 | A2 | 21-10-2004 | NONE | | |
| US 5245288 | A | 14-09-1993 | DE | 4126537 A1 | 11-02-1993 |
| | | | EP | 0527530 A1 | 17-02-1993 |
| | | | JP | 3187152 B2 | 11-07-2001 |
| | | | JP | H05261085 A | 12-10-1993 |
| | | | US | 5245288 A | 14-09-1993 |
| WO 2006067682 | A2 | 29-06-2006 | AT | 551615 T | 15-04-2012 |
| | | | CN | 101088021 A | 12-12-2007 |
| | | | EP | 1831710 A2 | 12-09-2007 |
| | | | JP | 4856094 B2 | 18-01-2012 |
| | | | JP | 2008523943 A | 10-07-2008 |
| | | | US | 2009237079 A1 | 24-09-2009 |
| | | | WO | 2006067682 A2 | 29-06-2006 |
| WO 2008001326 | A1 | 03-01-2008 | CN | 101479618 A | 08-07-2009 |
| | | | EP | 2038672 A1 | 25-03-2009 |
| | | | JP | 2009542295 A | 03-12-2009 |
| | | | US | 2009278537 A1 | 12-11-2009 |
| | | | WO | 2008001326 A1 | 03-01-2008 |
| US 2009058420 | A1 | 05-03-2009 | JP | 2009072580 A | 09-04-2009 |
| | | | US | 2009058420 A1 | 05-03-2009 |

EPO FORM P0459